# EUROPÄISCHE PATENTSCHRIFT

(11) **EP 3 448 293 B1**
(45) Veröffentlichungstag und Bekanntmachung des Hinweises auf die Patenterteilung: **03.06.2020**
(21) Anmeldenummer: 17720080.5
(22) Anmeldetag: 26.04.2017
(51) Int. Cl.: A61B 18/12, A61B 17/00, A61B 18/00

(54) **KUPPLUNG EINES ENDOSKOPISCHEN HF-CHIRURGISCHEN INSTRUMENTS UND HF-CHIRURGISCHES INSTRUMENT MIT EINER SOLCHEN KUPPLUNG**
COUPLING OF AN ENDOSCOPIC HF SURGICAL INSTRUMENT, AND HF SURGICAL INSTRUMENT COMPRISING SUCH A COUPLING
ACCOUPLEMENT D'UN INSTRUMENT CHIRURGICAL HF ENDOSCOPIQUE ET INSTRUMENT CHIRURGICAL HF MUNI D'UN TEL ACCOUPLEMENT

(30) Priorität: 27.04.2016 DE 102016207148; 28.06.2016 DE 102016211654
(43) Veröffentlichungstag der Anmeldung: 06.03.2019
(73) Patentinhaber: Endox Feinwerktechnik GmbH, 72581 Dettingen an der Erms (DE)
(72) Erfinder: HERNIK, Matthias, 72581 Dettingen/Erms (DE)
(74) Vertreter: Kordel, Mattias
(86) Internationale Anmeldenummer: PCT/EP2017/059867
(87) Internationale Veröffentlichungsnummer: WO 2017/186762

(56) Entgegenhaltungen:
- JP-U- H0 520 714
- US-A- 5 551 448
- US-A1- 2004 153 059
- US-A1- 2012 197 190
- US-A1- 2014 214 027

## Beschreibung

Die Erfindung betrifft eine Kupplung eines endoskopischen HF-chirurgischen Instruments gemäß Oberbegriff des Anspruchs 1 sowie ein HF-chirurgisches Instrument nach Anspruch 9.

HF-chirurgische Instrumente (hochfrequenzchirurgische Instrumente) zur Anwendung bei einer endoskopischen Submukosa Dissektion (Entfernung von Weichteilgewebe/Organen) (ESD) und einer endoskopischen Submukosa Resektion (Entfernung von bestimmten Gewebeteilen) (ESR) sind bekannt. Sie dienen zur Inzision in die Mukosa und zur Umschneidung und auch Entfernung eines pathologischen Bereichs in der Submukosa. Derartige Instrumente weisen typischerweise ein Schneidinstrument auf, welches an einem distalen Ende eines Katheters befestigt ist. Die Kopplung zwischen dem Schneidinstrument und dem Katheter ist häufig komplex, weil das Schneidinstrument derart mit dem Katheter gekoppelt werden muss, dass es sicher an den Einsatzort im Körper eines Patienten verlagerbar und wieder entfernbar ist, und dass eine Relativdrehung zwischen dem Katheter und dem Schneidinstrument gewährleistet ist. Um dies zu erreichen, werden spezielle Kupplungen verwendet, welche letztlich eine Selbstausrichtung von Instrumenten zum Gewebe ermöglichen und nicht - wie üblich - von Assistenzpersonal ausgerichtet werden müssen. Kupplungen dieser Art sind beispielsweise in Zusammenhang mit Polypektomieschlingen bekannt. Sie ermöglichen eine Relativdrehung zwischen einem Katheter und der Polypektomieschlinge (EP 1 691 695 B1). Die Montage derartiger Kupplungen ist, insbesondere bei kleinen Abmessungen des Katheters, aufwendig und umständlich. Dabei sind die Kupplungen höchst sicherheitsrelevant. Bei deren Verwendung mit HF-chirurgischen Instrumenten der oben genannten Art darf sich die Kopplung von Katheter und Schneidinstrument nicht lösen, weil sonst das Schneidinstrument nicht mehr ohne weiteres aus dem Operationsgebiet eines Patienten entfernt werden kann. Dies würde ein hohes gesundheitliches Risiko für diesen darstellen.

Dokument US 2014/214027 A1 offenbart eine Kupplung nach dem Oberbegriff des Anspruchs 1.

Aufgabe der Erfindung ist es daher, eine Kupplung eines endoskopischen HF-chirurgischen Instruments zu schaffen, welche derart ausgestaltet ist, dass die oben angesprochenen Nachteile vermieden werden.

Zur Lösung dieser Aufgabe wird eine Kupplung eines endoskopischen HF-chirurgischen Instruments mit den Merkmalen des Anspruchs 1 vorgeschlagen. Die Kupplung umfasst einen Grundkörper, welcher einen sich entlang einer Längsachse des Grundkörpers erstreckenden Hohlraum aufweist. In den Hohlraum ist ein Drehkörper bis zu einer Betriebsposition in einer Einführrichtung einführbar. Der Drehkörper ist in der Betriebsposition relativ zu dem Grundkörper um die Längsachse des Grundkörpers drehbar. Der Grundkörper weist eine Halteeinrichtung auf, welche eingerichtet ist, um den Drehkörper in der Betriebsposition in dem Grundkörper zu verrasten. Die Verrastung ist derart realisiert, dass eine Verlagerung des Drehkörpers relativ zu dem Grundkörper entlang der Längsachse blockierbar ist. Die Verrastung ist so ausgelegt, dass sie den Drehkörper nicht mehr freigibt. Es ist also nicht möglich, dass der Drehkörper ungewollt aus dem Grundkörper herausgezogen werden kann. Dabei wird davon ausgegangen, dass zumindest sichergestellt ist, dass die Entriegelung des Drehkörpers und damit dessen Freigabe ohne Lösemittel beziehungsweise spezielle Werkzeuge grundsätzlich nicht, zumindest nicht zerstörungsfrei erfolgen kann.

Der Grundkörper kann beispielsweise an einem distalen Ende eines Katheters angeordnet sein, an dessen proximalem Ende eine beispielsweise als Griff ausgebildete Manipulationseinrichtung angeordnet ist. Mit dem im Grundkörper verrasteten Drehkörper kann ein beispielsweise als Schneidinstrument ausgebildeter Effektor gekoppelt sein, welcher vorzugsweise in distaler Richtung aus dem Katheter ragt. Die Kupplung weist Vorteile gegenüber dem Stand der Technik auf: Bei herkömmlichen Kupplungen ist ein Grundkörper vorgesehen, der einen Innenraum umschließt. In diesen wird der Drehkörper eingeführt und dort in axialer Richtung gesichert. Diese Sicherung erfolgt durch Einbau von Anschlag- und Halteelementen, zwischen denen der Drehkörper in dem Hohlraum des Grundkörpers rotierbar gehalten wird. Die genannten Elemente werden in dem Grundkörper auf verschiedene Weise befestigt, beispielsweise durch Laserschweißen. Es ist also erforderlich, die Elemente in den Grundkörper in eine gewünschte Position einzuführen und dort zu fixieren. Wenn die Befestigung der Elemente in dem Grundkörper nicht mit höchster Präzision durchgeführt wird, ist es möglich, dass der Drehkörper in dem Grundkörper nicht rotieren kann, oder gar aus diesem herausrutscht, sodass eine Entkopplung zwischen dem Katheter und dem Schneidinstrument erfolgt. Daher muss die Montage der Kupplung mit höchster Präzision durchgeführt werden, was zeitaufwendig und kostenintensiv ist. Der Vorteil der erfindungsgemäßen Kupplung beruht darin, dass der Grundkörper eine Halteeinrichtung aufweist, mit welcher der Drehkörper mittels einer Rastverbindung verbunden werden kann. Allein durch Einführen des Drehkörpers in den Hohlraum des Grundkörpers kann eine sichere Fixierung des Drehkörpers derart erreicht werden, dass dieser zwar gegenüber dem Grundkörper drehbar bleibt, in axialer Richtung des Grundkörpers gesehen aber nicht mehr aus diesem versehentlich herausgezogen werden kann. Auf diese Weise ist es möglich, mit höchster Sicherheit zu gewährleisten, dass ein mit dem Katheter gekoppeltes Schneidinstrument nicht nur in den Körper eines Patienten einführbar, sondern auch wieder aus diesem entfernbar ist. Eine gesundheitliche Gefährdung des Patienten durch ein versehentlich vom Katheter abgetrenntes Schneidinstrument kann also praktisch ausgeschlossen werden.

Das HF-chirurgische Instrument ist insbesondere geeignet zur endoskopischen Submukosa Dissektion (ESD) oder Resektion (ESR) pathologischer Areale. Es ist vorzugsweise für endoskopische HF-chirurgische Operationen im menschlichen Körper geeignet.

Der Grundkörper ist vorzugsweise als hülsenförmiger Hohlzylinder mit - entlang der Längsachse gesehen - zwei gegenüber liegenden offenen Enden ausgebildet. Ein proximales Ende des Grundkörpers weist beispielsweise in Richtung eines Griffs. Ein - von dem proximalen Ende verschiedenes - distales Ende weist beispielsweise in Richtung eines Schneidinstruments.

Der Grundkörper wird vorzugsweise aus einem insbesondere nahtlosen Rohrelement hergestellt, welches zur Fertigstellung des Grundkörpers im Sinne der hier vorliegenden Erfindung mithilfe eines Lasers bearbeitet wird. Die Herstellung des einstückigen Grundkörpers ist daher einfach und kostengünstig. Das den Hohlzylinder bildende Rohrelement muss zur Herstellung des hier angesprochenen Grundkörpers lediglich einmal eingespannt und vorzugsweise einem einzigen Laserschneidvorgang unterworfen werden. Mittels eines Lasers wird aus dem den Grundkörper bildenden Rohrelement zur Realisierung einer Halteeinrichtung mindestens ein Schnitt in die Wandung des Rohrelements eingebracht, um das Drehelement in dem Grundkörper rotierbar derart sicher zu verrasten, dass es nicht mehr herausgezogen werden kann. Dazu werden aus der Wandung des Rohrelements mindestens ein Anschlag und wenigstens ein flexibles Halteelement herausgeschnitten. Es bedarf also nicht des Einbringens irgendwelcher Elemente in den Grundkörper, um den Drehkörper dort zu fixieren, wie dies bei herkömmlichen Kupplungen der Fall ist. Dadurch ist die Herstellung der Kupplung sehr kostengünstig und rasch durchführbar. Aufgrund der Tatsache, dass ein in sich geschlossenes Rohrelement als Rohrzylinder verwendet wird, ist der Grundkörper sehr stabil.

Der Grundkörper weist vorzugsweise wenigstens ein Metall oder wenigstens eine Metalllegierung auf, oder besteht aus einem Metall oder einer Metalllegierung. Alternativ oder zusätzlich kann der Grundkörper wenigstens einen Kunststoff aufweisen, oder aus einem Kunststoff bestehen.

Der in den Hohlraum des Grundkörpers einführbare Drehkörper ist vorzugsweise zylindrisch ausgebildet. Ein Außendurchmesser des Drehkörpers ist derart auf einen Innendurchmesser des Grundkörpers abgestimmt, dass der Drehkörper entlang der Längsachse in den Grundkörper einführbar und in diesem rotierbar ist. Eine Drehachse des Drehkörpers ist vorzugsweise deckungsgleich mit der Längsachse des Grundkörpers. Hier sei ausdrücklich darauf hingewiesen, dass der Hohlraum des Grundkörpers zwar vorzugsweise zylindrisch ausgebildet ist, dass aber die Außenfläche des Drehkörpers nicht zwingend ebenfalls zylindrisch sein muss. Denkbar ist es, dass die Außenfläche mindestens zwei Zylinderflächen aufweist, oder aber beispielsweise mehreckig ausgebildet ist, sodass unter anderem ein achteckiger Drehkörper realisiert wird, dessen Außenkanten vorzugsweise abgerundet sind und auf der Innenfläche des Hohlraums des Grundkörpers bei einer Rotation des Drehkörpers entlanggleiten. Wesentlich ist, dass der Drehkörper so ausgebildet ist, dass die Reibungskräfte zwischen Grund- und Drehkörper sehr gering sind und das Schneidinstrument gegenüber einer zu bearbeitenden Oberfläche des Körpers ausgerichtet bleibt, auch wenn der Katheter des HF-chirurgischen Instruments verdreht wird.

Es ist also möglich, dass der Grundkörper und der Drehkörper andersförmig ausgebildet sind. Entscheidend ist, dass eine Innenkontur des Grundkörpers auf eine Außenkontur des Drehkörpers derart abgestimmt ist, dass der Drehkörper in den Grundkörper einführbar und in diesem rotierbar ist.

Der Drehkörper weist - in Längsrichtung gesehen - ein proximales Ende und ein - davon verschiedenes - distales Ende auf. Der Drehkörper ist insbesondere mit seinem proximalen Ende an dem distalen Ende des Grundkörpers in diesen einführbar. Das distale Ende des Drehkörpers weist insbesondere in Richtung eines Schneidwerkzeugs.

Der Drehkörper ist in den Grundkörper bis zu der Betriebsposition in der Einführrichtung einführbar. Die Betriebsposition bezeichnet diejenige Position des Drehkörpers in dem Grundkörper, welche der Drehkörper insbesondere während eines Betriebs oder einer Verwendung des endoskopischen HF-chirurgischen Instruments, beispielsweise einer Operation, einnimmt. Die Betriebsposition stellt insbesondere eine Lage des Drehkörpers in dem Grundkörper dar, welche hinsichtlich einer - entlang der Längsachse gesehen - axialen und radialen Position des Drehkörpers relativ zu dem Grundkörper im Wesentlichen festgelegt ist.

Die Einführrichtung bezeichnet insbesondere die Richtung einer Verlagerung, welche - entlang der Längsachse - durch einen von dem distalen Ende des Grundkörpers zu dem proximalen Ende desselben zeigenden Vektors definiert wird.

Der Drehkörper ist in der Betriebsposition relativ zu dem Grundkörper um die Längsachse drehbar. Vorzugsweise ist der Drehkörper relativ zu dem Grundkörper mit wenig Spiel, besonders bevorzugt spielfrei drehbar. Die Reibung bei der Relativdrehung des Drehkörpers ist vorzugsweise derart gering, dass ein mit dem Drehkörper gekoppeltes Schneidinstrument, beispielsweise eine HF-Schneidnadel, sich dem zu behandelnden Gewebe an einer Eingriffsstelle im Körper eines Patienten hinsichtlich einer Drehwinkellage relativ zu dem Grundkörper anpassen und zu diesem ausrichten kann.

Mittels der Halteeinrichtung ist der Grundkörper mit dem Drehkörper insbesondere in der Betriebsposition formschlüssig koppelbar. Dabei kann die Verlagerung des Drehkörpers relativ zu dem Grundkörper aus der Betriebsposition entlang der Längsachse mit wenig Spiel, besonders bevorzugt spielfrei gehalten werden. Zusätzlich ist die formschlüssige Kopplung vorzugsweise derart ausgebildet, dass die Drehung des Drehkörpers relativ zu dem Grundkörper reibungsarm möglich ist.

Das endoskopische HF-chirurgische Instrument ist vorzugsweise flexibel ausgebildet. Besonders bevorzugt ist das endoskopische HF-chirurgische Instrument geeignet, in einem Arbeitskanal eines Endoskops geführt zu werden. Der Arbeitskanal ermöglicht beispielsweise ein Einführen des Instruments in den menschlichen Körper, um ein Schneidinstrument an der Eingriffsstelle im Körper des Patienten zu positionieren. Es ist insbesondere auch möglich, dass das endoskopische HF-chirurgische Instrument nur zum Teil flexibel ausgebildet ist. Es ist auch möglich, dass das endoskopische HF-chirurgische Instrument starr ausgebildet ist.

Es wird ein Ausführungsbeispiel der Kupplung bevorzugt, das sich dadurch auszeichnet, dass der Drehkörper in der Betriebsposition gänzlich in dem Hohlraum angeordnet ist.

Es wird ein Ausführungsbeispiel der Kupplung bevorzugt, das sich dadurch auszeichnet, dass die Halteeinrichtung wenigstens ein erstes Halteelement aufweist, welches in dem Hohlraum einen Anschlag ausbildet. Der Anschlag wirkt in der Betriebsposition mit dem Drehkörper derart zusammen, dass eine Verlagerung desselben, in der Einführrichtung gesehen, über die Betriebsposition hinaus verhindert wird.

Bei einer erfindungsgemäßen Kupplung ist das wenigstens eine erste Halteelement einstückig mit dem Grundkörper ausgebildet. Das erste Halteelement kann als Lasche ausgebildet sein. Vorzugsweise ist in einer Mantelfläche des Grundkörpers ein im Wesentlichen U-förmiger Schnitt, beispielsweise mittels Laserschneiden, eingebracht, wobei sich das "U" in Richtung des proximalen Endes des Grundkörpers öffnet. Es ergibt sich auf diese Weise eine Lasche, welche vorzugsweise in den Hohlraum hineingebogen werden kann. Vorzugsweise ist die Lasche im Bereich eines freien Endes derselben im Wesentlichen orthogonal zu der Längsachse ausgerichtet.

Der Anschlag wirkt in der Betriebsposition vorzugsweise mit dem proximalen Ende des Drehkörpers zusammen. Der Anschlag wirkt insbesondere in der Betriebsposition mit dem Drehkörper derart zusammen, dass verhindert wird, dass der Drehkörper über die Betriebsposition hinaus entlang der Längsachse in der Einführrichtung verlagerbar ist. Besonders bevorzugt ist das mindestens eine erste Halteelement nicht-federnd ausgebildet. Auf diese Weise wird eine sehr exakte axiale Position des Drehkörpers in dem Grundkörper realisiert.

Es wird ein Ausführungsbeispiel der Kupplung bevorzugt, das sich dadurch auszeichnet, dass die Halteeinrichtung wenigstens ein zweites Halteelement aufweist, welches zum Einführen des Drehkörpers in den Hohlraum gegen eine Rückstellkraft in eine Freigabestellung verlagerbar ist. Vorzugsweise weist die Halteeinrichtung zwei zweite Halteelemente auf. Das mindestens eine zweite Halteelement ist flexibel ausgebildet. Damit wird Folgendes ausgesagt: Das Halteelement muss nicht über seine gesamte Länge flexibel sein. Es reicht, wenn es mit einem Ende flexibel mit dem Grundkörper derart verbunden ist, dass es gegenüber diesem gegen eine Rückstellkraft verlagerbar ist. Das mindestens eine zweite Halteelement wird, wie gesagt, durch einen U-förmigen Schnitt, insbesondere Laser-Schnitt, aus dem Grundkörper herausgeschnitten, sodass es eine U-förmige Außenkontur aufweist. Das freie Ende wird in das Innere des Grundkörpers so hineingebogen, dass es einen spitzen Winkel gegenüber der Längsachse des Grundkörpers einnimmt und mit dem dem freien Ende gegenüberliegenden Ende in die Wandung des Grundkörpers übergeht. Dieser Übergangsbereich ist so ausgestaltet, dass das mindestens eine zweite Halteelement aus der Grundstellung, in welcher es einen spitzen Winkel mit der Längsachse einschließt, nach außen federnd gebogen werden kann, wie unten erläutert ist. Denkbar ist es auch, dass lediglich Bereiche des mindestens einen zweiten Halteelements federnd elastisch ausgebildet sind.

Bei einer erfindungsgemäßen Kupplung ist das wenigstens eine zweite Halteelement einstückig mit dem Grundkörper ausgebildet. Das wenigstens eine zweite Halteelement kann als rastnasenförmige Lasche ausgebildet sein. Vorzugsweise weist der Grundkörper einen, beispielsweise mittels Laserschneiden hergestellten U-förmigen Schnitt in der Mantelfläche auf, wobei sich das "U" in Richtung des distalen Endes des Grundkörpers öffnet. Durch Umbiegen der dadurch erzeugten Lasche in Richtung des Hohlraums kann somit eine Rastnase realisiert werden, die in den Hohlraum hineinragt. Die rastnasenförmige Lasche ist insbesondere an einem, einem freien Ende derselben gegenüberliegenden Bereich biegsam an der Mantelfläche des Grundkörpers angelenkt.

Das wenigstens eine zweite Halteelement ist zum Einführen des Drehkörpers in den Hohlraum gegen die Rückstellkraft in die Freigabestellung verlagerbar. Vorzugsweise wird dabei das wenigstens eine zweite Halteelement nach außen, also entlang der Längsachse gesehen - radial nach außen verlagert, insbesondere verschwenkt. Besonders bevorzugt wird die Verlagerung desselben in die Freigabestellung durch den Drehkörper beim Einführen desselben in den Grundkörper bewirkt. Der Drehkörper drückt dann quasi das wenigstens eine zweite Halteelement radial nach außen, sodass ein Querschnitt des Hohlraums im Bereich des wenigstens einen zweiten Halteelements derart aufgeweitet wird, dass der Drehkörper in die Betriebsposition verlagert werden kann.

Bei einem anderen bevorzugten Ausführungsbeispiel wird die Verlagerung des Drehkörpers in der Einführrichtung über die Betriebsposition hinaus dadurch verhindert, dass ein Anschlag an dem Drehkörper selbst ausgebildet ist. Es ist auch möglich, dass eine derartige Begrenzung zur Verhinderung einer über die Betriebsposition hinausreichende Verlagerung des Drehkörpers relativ zu dem Grundkörper auf andere Weise realisiert wird. Entscheidend ist, dass eine derartige Begrenzung realisiert ist.

Es wird ein Ausführungsbeispiel der Kupplung bevorzugt, das sich dadurch auszeichnet, dass die Rückstellkraft eine Verlagerung des wenigstens einen zweiten Halteelements in eine Haltestellung bewirkt, wenn sich der Drehkörper in der Betriebsposition befindet. Dadurch ist vorzugsweise eine Verlagerung des Drehkörpers entgegen der Einführrichtung verhindert.

Die Haltestellung unterscheidet sich insbesondere dadurch von der Freigabestellung, dass das wenigstens eine zweite Halteelement radial in den Hohlraum verlagert, beispielsweise hineingebogen ist. Insbesondere rastet das wenigstens eine zweite Halteelement quasi - in Einführrichtung gesehen - hinter dem distalen Ende des Drehkörpers ein, wenn dieser die Betriebsposition erreicht hat.

Es ist insbesondere nicht möglich, durch Ziehen des Drehkörpers entgegen der Einführrichtung das wenigstens eine zweite Halteelement ohne weiteres aus der Haltestellung in die Freigabestellung zu drängen. Eine ungewünschte Verlagerung des Drehkörpers aus der Betriebsposition entgegen der Einführrichtung, also eine Entkopplung von Grundkörper und Drehkörper wird auf diese Weise mit höchster Sicherheit verhindert.

Es wird ein Ausführungsbeispiel der Kupplung bevorzugt, das sich dadurch auszeichnet, dass eine Verlagerung des wenigstens einen zweiten Halteelements in die Freigabestellung durch ein Lösemittel bewirkbar ist. Dadurch kann der Drehkörper vorzugsweise aus der Betriebsposition entgegen der Einführrichtung verlagert werden.

Das Lösemittel ist vorzugsweise eingerichtet, um mit dem wenigstens einen zweiten Halteelement derart zusammenzuwirken, dass eine Verlagerung desselben in die Freigabestellung bewirkt wird. Vorzugsweise ist das Lösemittel eingerichtet, um mittels Aufbringen einer Druckkraft auf das wenigstens eine zweite Halteelement eine Verlagerung desselben in radialer Richtung nach außen in die Freigabestellung zu bewirken.

Das Lösemittel kann einstückig mit dem Grundkörper ausgebildet sein. Vorzugsweise ist das Lösemittel jedoch ein von dem Grundkörper separiertes Teil. Es ist möglich, dass das Lösemittel unverlierbar an dem Grundkörper oder einem anderen Teil des endoskopischen HF-chirurgischen Instruments angeordnet ist.

Es kann mithin eine sichere Verrastung und Fixierung des Drehkörpers in dem Grundkörper in der Betriebsposition bewirkt werden, indem ein Freigeben einer Verlagerung des Drehkörpers entgegen der Einführrichtung vorzugsweise nur mittels des Lösemittels oder eines sonstigen Werkzeugs möglich ist.

Es wird ein Ausführungsbeispiel der Kupplung bevorzugt, das sich dadurch auszeichnet, dass die Verlagerung des wenigstens einen zweiten Halteelements in die Freigabestellung durch Einführen des Lösemittels in den Hohlraum bewirkbar ist.

Bei einem bevorzugten Ausführungsbeispiel der Kupplung ist das Lösemittel als zylindrische Hülse ausgebildet, welche vorzugsweise einen in einer Längsrichtung derselben angeordneten Schlitz aufweist. Eine Breite des Schlitzes ist vorzugsweise so gewählt, dass die Hülse auf einen, mit dem Drehkörper gekoppelten Schaft, beispielsweise eines Schneidinstruments aufgesetzt werden kann, indem der Schaft durch den Schlitz hindurchgeführt wird, sodass das Lösemittel den Schaft zumindest abschnittsweise umschließt.

Das Lösemittel ist vorzugsweise entlang der Einführrichtung auf dem Schaft in Richtung der Betriebsposition verlagerbar, so dass es bei einer bestimmten Position in Eingriff mit dem wenigstens einen flexiblen zweiten Halteelement gebracht werden kann. Bei einem weiteren Verlagern des Lösemittels in Einführrichtung in Richtung des Drehkörpers wirkt das Lösemittel derart mit dem wenigstens einen zweiten Halteelement zusammen, dass es aus der Haltestellung nach außen in die Freigabestellung verlagert werden kann. Das wenigstens eine zweite Halteelement kann somit durch Einführen des Löseelements in den Hohlraum insbesondere radial nach außen gedrückt werden, sodass ein Ausfahren des Drehkörpers aus dem Grundkörper ermöglicht wird.

Bei einem bevorzugten Ausführungsbeispiel der Kupplung ist ein Außendurchmesser des Lösemittels gleich dem Außendurchmesser des Drehkörpers ausgebildet. In diesem Fall kann nach Verlagerung des wenigstens einen zweiten Halteelements in die Freigabestellung der Drehkörper zusammen mit dem Lösemittel entgegen der Einführrichtung aus dem Hohlraum ausgefahren werden.

Bei einem anderen bevorzugten Ausführungsbeispiel ist der Außendurchmesser des Drehkörpers kleiner als ein Innendurchmesser des Lösemittels, so dass der Drehkörper durch das Lösemittel hindurch verlagert werden kann. In diesem Fall wird das Lösemittel in den Hohlraum insbesondere in Einführrichtung eingefahren, wobei es eine Verlagerung des wenigstens einen zweiten Halteelements von der Haltestellung nach außen in die Freigabestellung bewirkt. Der Drehkörper kann dann entgegen der Einführrichtung aus der Betriebsposition verlagert werden, wobei er vorzugsweise axial durch das hülsenförmige Lösemittel geschoben oder gezogen wird. Es ist also insbesondere möglich, dass das Lösemittel nach Ausfahren des Drehkörpers in dem Hohlraum verbleibt, beispielsweise bis ein anderes Schneidwerkzeug mittels gekoppeltem Drehkörper in den Grundkörper eingeführt ist. Dies kann vorteilhaft sein, da so vorzugsweise beim Einführen des Drehkörpers keine Kraft zum Verlagern des wenigstens einen zweiten Halteelements in die Freigabestellung erforderlich ist.

Es ist auch möglich, dass das Lösemittel anders ausgebildet ist. Es ist ebenso möglich, dass das Lösemittel in einer von der Einführrichtung verschiedenen Richtung in den Grundkörper einführbar ist.

Es wird ein Ausführungsbeispiel der Kupplung bevorzugt, das sich dadurch auszeichnet, dass mit dem Drehkörper ein Effektor, vorzugsweise ein Schneidinstrument, insbesondere ein als Schneidinstrument ausgebildeter Effektor, gekoppelt ist. Vorzugsweise ist das Schneidinstrument als Schneidnadel, besonders bevorzugt als HF-Schneidnadel ausgebildet. Es ist aber auch möglich, dass das Schneidinstrument als Messer oder in einer anderen Form ausgebildet ist.

Bei einem bevorzugten Ausführungsbeispiel der Kupplung ist das Schneidinstrument als HF-chirurgisches Schneidinstrument ausgebildet. Es ist vorzugsweise zu einem zu schneidenden Gewebe im Körper des Patienten selbstausrichtend ausgebildet. Insbesondere ist es möglich, dass sich das Schneidinstrument unabhängig von einer Drehwinkellage oder Drehbewegung des Grundkörpers um dessen Längsachse drehen kann. Insoweit kann eine Selbstausrichtung insbesondere unabhängig von einer Drehwinkellage oder Drehbewegung des Griffs des endoskopischen HF-chirurgischen Instruments erfolgen.

Bei einem bevorzugten Ausführungsbeispiel der Kupplung ist das Schneidinstrument als HF-chirurgisches Schneidinstrument ausgebildet, wobei der Drehkörper insbesondere an seinem distalen Ende mit dem Schaft des Schneidinstruments gekoppelt ist, welcher mindestens einen Draht aufweist. Vorzugsweise weist dieser einen Durchmesser von ca. 0,2 mm bis ca. 1,0 mm, vorzugsweise von ca. 0,4 mm bis ca. 0,8 mm, insbesondere 0,6 mm auf. Der hier angesprochene Schaft des Schneidinstruments kann auch wenigstens zwei Drähte aufweisen, die ca. 0,2 mm bis ca. 0,5 mm, vorzugsweise ca. 0,3 mm bis 0,4 mm dick sind. Die beiden Drähte sind miteinander in Abständen vorzugsweise stoffschlüssig verbunden, beispielsweise durch Laserpunktschweißen.

Es wird ein Ausführungsbeispiel der Kupplung bevorzugt, das sich dadurch auszeichnet, dass der Grundkörper in einem Katheter angeordnet ist. Der Katheter ist vorzugsweise hohl, insbesondere als Schlauch ausgebildet. Vorzugsweise ist der Katheter flexibel ausgebildet. Es ist aber auch möglich, dass der Katheter teilweise flexibel oder starr ausgebildet ist. Besonders bevorzugt weist der Katheter Polytetrafluorethen (PTFE), auch als Teflon bezeichnet, auf, oder besteht aus diesem Material. Der Katheter ist vorzugsweise elektrisch isolierend ausgebildet.

An einem proximalen Ende des Katheters ist vorzugsweise ein Griff oder ein Manipulationsbereich vorgesehen, welcher insbesondere der Steuerung einer axialen Bewegung des insbesondere in einem Arbeitskanal eines Endoskops geführten Katheters dient. Mit axialer Bewegung ist eine Bewegung in Richtung der Erstreckung des Katheters gemeint. Im Bereich eines distalen Endes des Katheters ist der Grundkörper der Kupplung in dem Katheter angeordnet. Der Grundkörper ist vorzugsweise fest in dem Katheter eingepresst, wobei der Grundkörper insbesondere verschiebesicher, gegebenenfalls auch verdrehsicher in dem Katheter gehalten ist. Durch den lagefesten Einbau des Grundkörpers der Kupplung folgt dieser allen Bewegungen des Endes des Katheters sowohl in axialer Richtung als auch bei seitlichen Bewegungen des Katheterendes. Damit folgt auch das endoskopische chirurgische Schneidinstrument, welches der Kupplung zugeordnet ist, diesen Bewegungen. Der Grundkörper der Kupplung kann gegebenenfalls eine Rotation um die Längsachse des Katheters durchführen. Dieser Drehbewegung folgt allerdings das endoskopische chirurgische Schneidinstrument nicht, weil dieses mit dem drehbar im Grundkörper gelagerten Drehkörper gekoppelt, also von dem Grundkörper und dem Katheter bezüglich Drehbewegungen entkoppelt ist.

Bei einem bevorzugten Ausführungsbeispiel der Kupplung weist der Katheter im Bereich des distalen Endes auf einer Innenseite einen Innendurchmesser von ca. 1,0 mm bis ca. 3,0 mm, vorzugsweise von ca. 1,5 mm bis 1,6 mm auf. Der in dem Katheter angeordnete Grundkörper weist vorzugsweise einen Außendurchmesser von ca. 1,1 mm bis ca. 3,1 mm, vorzugsweise von ca. 1,7 mm auf. Der Katheter ist zumindest im Bereich seines distalen Endes in radialer Richtung flexibel ausgebildet, so dass auf einfache Weise durch Kraftschluss eine verschiebesichere und gegebenenfalls auch eine verdrehsichere Fixierung des Grundkörpers in dem Katheter realisiert werden kann. Es ist insbesondere möglich, dass der Grundkörper auf einer Außenseite desselben, zumindest bereichsweise bearbeitet, vorzugsweise sandgestrahlt ist, um den Kraftschluss mit dem Katheter weiter zu verbessern.

Bei einem bevorzugten Ausführungsbeispiel der Kupplung kragt das distale Ende des elektrisch isolierten Katheters mit einer Länge von 5 mm bis 15 mm, vorzugsweise von 10 mm über das distale Ende des Grundkörpers hinaus.

Es wird ein Ausführungsbeispiel der Kupplung bevorzugt, das sich dadurch auszeichnet, dass der Katheter einen Stromleiter aufweist, mit welchem der Grundkörper elektrisch verbunden ist. Der Stromleiter ist insbesondere eingerichtet, um elektrische Energie, welche zum Betrieb von HF-chirurgischen Instrumenten erforderlich ist, von einer Stromquelle bis zu dem Grundkörper zu führen.

Bei einem bevorzugten Ausführungsbeispiel der Kupplung ist der Stromleiter schraubenfederförmig ausgebildet und vorzugsweise an der Innenseite des Katheters dergestalt angeordnet, dass ein durch den Stromleiter gebildeter lichter Querschnitt auf die Außenmaße des Grundkörpers derart abgestimmt ist, dass der Grundkörper in einem durch den schraubenfederförmigen Stromleiter ausgebildeten Raum angeordnet werden kann. Vorzugsweise weist der Grundkörper einen elektrischen Kontakt auf, welcher mit dem Stromleiter elektrisch verbunden ist.

Alternativ oder zusätzlich ist der Stromleiter bandförmig ausgebildet. Vorzugsweise ist an der Innenseite des Katheters wenigstens ein stromleitendes Band angeordnet. Dieses ist vorzugsweise in die Innenseite desselben eingelassen. Bevorzugt ist das wenigstens eine Band aus Metallfolie ausgebildet. Es ist aber auch möglich, dass das wenigstens eine Band leitenden Kunststoff aufweist oder aus leitendem Kunststoff besteht. Ein derartiger Katheter kann beispielsweise mittels Zwei-Komponenten-Extrusion realisiert werden. Vorzugsweise weist der Grundkörper einen elektrischen Kontakt auf, welcher mit dem Stromleiter elektrisch verbunden ist.

Alternativ oder zusätzlich ist der Stromleiter drahtförmig, vorzugsweise litzenförmig ausgebildet. Vorzugsweise ist ein Draht, besonders bevorzugt eine Litze insbesondere lose im Inneren des Katheters angeordnet. Der Draht ist vorzugsweise an einer geeigneten Stelle des Grundkörpers, besonders bevorzugt einem im Bereich des proximalen Endes angeordneten Schlitz verbunden, beispielsweise mittels Laserschweißen.

Alternativ oder zusätzlich ist der Stromleiter geflechtartig ausgebildet. Vorzugsweise ist das Geflecht zwischen der Innenseite und einer Außenseite des Katheters angeordnet. Der geflechtartige Stromleiter ist vorzugsweise als "braided tube" ausgebildet, wobei er sich rohrförmig entlang des Katheters erstreckt. Der Grundkörper weist vorzugsweise wenigstens eine scharfe Kante an der Außenseite desselben auf, welche eingerichtet ist, in den Katheter über die Innenseite desselben soweit einzudringen, dass ein elektrischer Kontakt zwischen der Kante und dem geflechtartigen Stromleiter herstellbar ist.

Insbesondere durch die schraubenfederförmige, bandförmige oder geflechtartige Ausbildung des Stromleiters ist eine einfache Montage des Grundkörpers in dem Katheter möglich, da bei einem Einführen des Grundkörpers in den Katheter einfach, vorzugsweise automatisch, ein elektrischer Kontakt zwischen dem Grundkörper und dem Stromleiter hergestellt wird. Die drahtförmige Ausbildung des Stromleiters stellt aufgrund ihrer besonders einfachen Ausgestaltung eine besonders kostengünstige Lösung dar.

Es wird ein Ausführungsbeispiel der Kupplung bevorzugt, das sich dadurch auszeichnet, dass der Grundkörper und der Drehkörper, und/oder der Drehkörper und das Schneidinstrument elektrisch miteinander verbunden sind. Eine Innenseite des Grundkörpers ist vorzugsweise im Bereich der Betriebsposition zumindest bereichsweise elektrisch leitfähig ausgebildet. Der Drehkörper ist vorzugsweise an einer Mantelfläche desselben zumindest bereichsweise elektrisch leitfähig ausgebildet. Es ist somit insbesondere möglich, eine unterbrechungsfreie elektrische Verbindung zwischen dem Grundkörper und dem Drehkörper bei einem bestimmungsgemäßen Betrieb des endoskopischen HF-chirurgischen Instruments sicherzustellen. Besonders bevorzugt kann selbst bei einem kurzzeitig freischwebenden Zustand des Drehkörpers in dem Grundkörper eine elektrische Verbindung des Drehkörpers mit dem Grundkörper realisiert werden, indem beispielsweise durch Plasmabildung oder Funkenübertragung eine elektrische Verbindung an wenigstens einer Stelle realisiert wird.

Der Drehkörper ist vorzugsweise derart mit dem Schneidinstrument gekoppelt, dass eine sichere Übertragung elektrischer Energie, welche insbesondere bei HF-chirurgischen Anwendungen erforderlich ist, übertragen werden kann. Vorzugsweise erfolgt die Energieübertragung über einen elektrisch leitfähigen Schaft.

Es ist also insbesondere möglich, eine Stromquelle über den Stromleiter, den Grundkörper, den Drehkörper mit dem Schneidinstrument elektrisch zu verbinden.

Zur Lösung der genannten Aufgabe wird außerdem ein HF-chirurgisches Instrument vorgeschlagen, welches die in Anspruch 9 genannten Merkmale aufweist, also einen Katheter umfasst, wie er oben erläutert wurde. Es ist insbesondere möglich, dass der Katheter flexibel ausgebildet ist. Es ist aber auch möglich, dass der Katheter teilweise flexibel oder starr ausgebildet ist.

Es wird ein Ausführungsbeispiel des HF-chirurgischen Instruments bevorzugt, das sich dadurch auszeichnet, dass das HF-chirurgische Instrument flexibel ausgebildet ist. Besonders bevorzugt ist das endoskopische HF-chirurgische Instrument geeignet, in einem Arbeitskanal eines Endoskops geführt zu werden. Der Arbeitskanal ermöglicht beispielsweise ein Einführen des Instruments in den menschlichen Körper, um ein Schneidinstrument an der Eingriffsstelle im Körper des Patienten zu positionieren.

Es wird ein Ausführungsbeispiel des HF-chirurgischen Instruments bevorzugt, das sich dadurch auszeichnet, dass ein Katheter vorgesehen ist, in welchem die Kupplung verschiebesicher anordenbar ist.

Es wird ein Ausführungsbeispiel des HF-chirurgischen Instruments bevorzugt, das sich dadurch auszeichnet, dass der Katheter ein distales Ende aufweist, in dessen Bereich die Kupplung anordenbar ist.

Die Erfindung wird im Folgenden anhand der Zeichnung näher erläutert. Dabei zeigen:
- Figur 1: ein Ausführungsbeispiel einer Kupplung eines endoskopischen HF-chirurgischen Instruments in einem entkoppelten Zustand a) in einer perspektivischen Darstellung, b) in einer Draufsicht und c) in einem Längsschnitt;
- Figur 2: das Ausführungsbeispiel der Kupplung in einem gekoppelten Zustand in einem Längsschnitt;
- Figur 3: eine schematische Darstellung eines Ausführungsbeispiels der Kupplung mit einem Lösemittel a) in einem Längsschnitt, sowie b) das Lösemittel in einer perspektivischen Darstellung;
- Figur 4: eine schematische Darstellung eines ersten Ausführungsbeispiels der Kupplung mit einem Stromleiter in einem Längsschnitt;
- Figur 5: eine schematische Darstellung eines zweiten Ausführungsbeispiels der Kupplung mit einem Stromleiter in einem Längsschnitt;
- Figur 6: eine schematische Darstellung eines dritten Ausführungsbeispiels der Kupplung mit einem Stromleiter in einem Längsschnitt;
- Figur 7: eine schematische Darstellung eines vierten Ausführungsbeispiels der Kupplung mit einem Stromleiter in einem Längsschnitt.

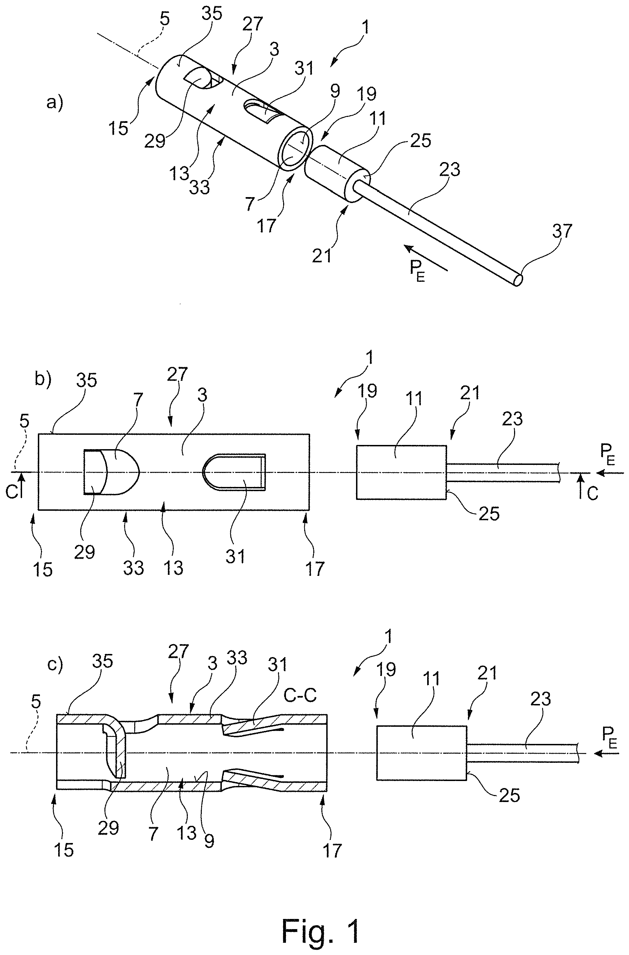

Figur 1a) zeigt ein Ausführungsbeispiel einer Kupplung 1 eines endoskopischen HF-chirurgischen Instruments in einem entkoppelten Zustand in einer perspektivischen Darstellung. Die Kupplung 1 weist einen Grundkörper 3 auf, welcher im Wesentlichen als hülsenförmiger Hohlzylinder ausgebildet ist. Der Grundkörper 3 weist einen sich entlang einer Längsachse 5 des Grundkörpers 3 erstreckenden Hohlraum 7 auf. Der Hohlraum 7 wird im Wesentlichen durch eine Innenseite 9 des Grundkörpers 3 in radialer Richtung begrenzt. In den Hohlraum 7 ist ein Drehkörper 11 bis zu einer Betriebsposition 13 in einer Einführrichtung einführbar. Die Einführrichtung ist durch einen Pfeil P_{E} gekennzeichnet. Das Einführen des Drehkörpers 11 in den Grundkörper 3 erfolgt in der Einführrichtung P_{E} entlang der Längsachse 5 des Grundkörpers 3. Bei dem in Figur 1a) dargestellten Ausführungsbeispiel fällt eine Längsachse des Drehkörpers 11 mit der Längsachse 5 des Grundkörpers 3 zusammen.

Die Betriebsposition 13 entspricht einer Position des Drehkörpers 11 in dem Grundkörper 3 bei bestimmungsgemäßem Betrieb des endoskopischen HF-chirurgischen Instruments, beispielsweise bei einer Operation. Die Betriebsposition 13 befindet sich in einem Bereich des Grundkörpers 3 zwischen einem proximalen Ende 15 des Grundkörpers 3 und einem distalen Ende 17 des Grundkörpers 3. Der Drehkörper 11 weist ein proximales Ende 19 und ein distales Ende 21 auf. Beim Einführen des Drehkörpers 11 in den Grundkörper 3 dringt der Drehkörper 11 mit dem proximalen Ende 19 zuerst in den Hohlraum 7 an dem distalen Ende 17 des Grundkörpers 3 ein. In der Betriebsposition 13 ist der Drehkörper 11 vorzugsweise vollständig von dem Grundkörper 3 umschlossen.

Bei einem bevorzugten Ausführungsbeispiel der Kupplung 1 ist - aus Sicht eines Betrachters gesehen - links von dem Grundkörper 3 ein hier nicht dargestellter Griff des endoskopischen HF-chirurgischen Instruments angeordnet. Der Griff ist mit dem Grundkörper 3 beispielsweise über einen ebenfalls nicht wiedergegebenen Katheter gekoppelt. Vorzugsweise ist der Drehkörper 11 mit einem Schneidwerkzeug gekoppelt, welches - aus Sicht des Betrachters gesehen - rechts von dem Drehkörper 11 angeordnet und hier nicht dargestellt ist. Der Drehkörper 11 ist mit einem Schneidwerkzeug vorzugsweise über einen Schaft 23 koppelbar. Der Schaft 23 ist vorzugsweise form-, kraft- oder stoffschlüssig mit dem Drehkörper 11 verbunden, wobei er hier an dessen distalem Ende 21 entspringt.

Der Drehkörper 11 weist an dem distalen Ende 21 einen vorzugsweise plan ausgebildeten distalen Abstützbereich 25 auf. Der distale Abstützbereich 25 liegt vorzugsweise in einer Ebene, welche orthogonal zu der Längsachse 5 angeordnet ist.

Der Grundkörper 3 weist eine Halteeinrichtung 27 auf. Die Halteeinrichtung 27 ist eingerichtet, um den Grundkörper 3 mit dem Drehkörper 11 in der Betriebsposition 13 formschlüssig derart zu verrasten, dass eine Verlagerung des Drehkörpers 11 relativ zu dem Grundkörper 3 entlang der Längsachse 5 blockierbar ist. Die Halteeinrichtung 27 weist hierzu wenigstens ein erstes Halteelement 29 und wenigstens ein zweites Halteelement 31 auf. Das wenigstens eine erste Halteelement 29 und das wenigstens eine zweite Halteelement 31 sind hier als Laschen ausgebildet. Die Laschen können beispielsweise mittels eines Laserstrahls aus einem Grundkörpermantel 33 ausgeschnitten werden, wobei der Laserstrahl in eine Außenseite 35 des Grundkörpers 3 eingeleitet wird. Die Laschen sind vorzugsweise in den Hohlraum 7 hineingebogen. Der Drehkörper 11 ist durch die Halteeinrichtung 27 derart lagerbar, dass er in der Betriebsposition 13 relativ zu dem Grundkörper 3 um die Längsachse 5 drehbar ist.

Vorzugsweise handelt es sich bei der Kupplung 1 um eine Kupplung eines endoskopischen HF-chirurgischen Instruments, welches flexibel ausgebildet ist. Es ist also insbesondere möglich, das endoskopische HF-chirurgische Instrument in einem Arbeitskanal eines Endoskops zu einer Eingriffsstelle im Körper eines Patienten zu führen. In diesem Fall ist beispielsweise der Grundkörper 3 mit einem in dem Arbeitskanal verlagerbaren Katheter insbesondere verschiebesicher und gegebenenfalls verdrehsicher gekoppelt, wobei der Katheter geeignet ist, den Grundkörper 3 sowie einen mit diesem gekoppelten Drehkörper 11 in Richtung der Eingriffsstelle oder von dieser weg zu führen.

Besonders bevorzugt ist der Drehkörper 11 mit einem Schneidinstrument gekoppelt, welches über den Schaft 23 mit demselben verbunden ist. Besonders bevorzugt ist das Schneidinstrument als HF-chirurgisches Schneidinstrument ausgebildet. Das Schneidinstrument ist in Figur 1a) nicht dargestellt. Es ist vorzugsweise an einem - hier nur angedeuteten - distalen Ende 37 des Schafts 23 angeordnet. Es ist möglich, dass der Schaft 23 und das Schneidinstrument einstückig ausgebildet sind. Der Schaft 23 weist mindestens einen Draht auf, welcher an dem distalen Ende 37 des Schafts 23 das Schneidinstrument ausbildet. Besonders bevorzugt handelt es sich bei dem Schneidinstrument um eine Schneidnadel. Es ist sehr wohl möglich, den Schaft 23 mit mindestens zwei Drähten auszubilden.

Figur 1b) zeigt das Ausführungsbeispiel der Kupplung 1 in dem entkoppelten Zustand in einer Draufsicht. Diese Draufsicht ist im Wesentlichen - aus Sicht des Betrachters - eine Sicht von oben auf Figur 1a). Gleiche und funktionsgleiche Elemente sind mit gleichen Bezugszeichen versehen, sodass insofern auf die vorangegangene Beschreibung verwiesen wird.

Figur 1c) zeigt das Ausführungsbeispiel der Kupplung 1 in dem entkoppelten Zustand in einem Längsschnitt entlang der Linie C-C gemäß Figur 1b). Gleiche und funktionsgleiche Elemente sind mit gleichen Bezugszeichen versehen, sodass insofern auf die vorangegangene Beschreibung verwiesen wird.

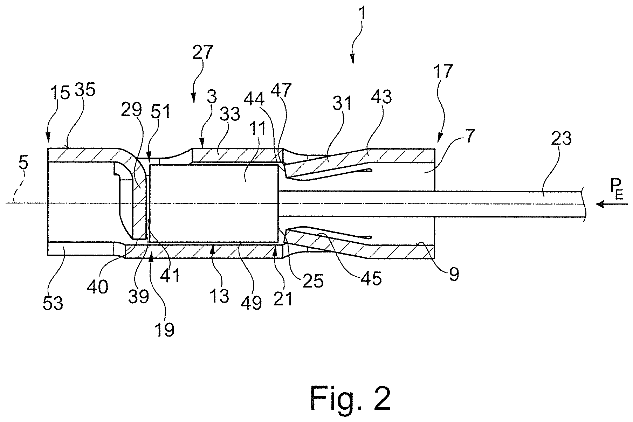

Figur 2 zeigt in einem Längsschnitt das Ausführungsbeispiel der Kupplung 1 in einem Zustand, in welchem der Grundkörper 3 und der Drehkörper 11 in der Betriebsposition 13 miteinander gekoppelt sind. Gleiche und funktionsgleiche Elemente sind mit gleichen Bezugszeichen versehen, sodass insofern auf die vorangegangene Beschreibung verwiesen wird.

Der Drehkörper 11 ist hier somit in der Betriebsposition 13 gänzlich in dem Hohlraum 7 angeordnet.

Die Halteeinrichtung 27 weist das mindestens eine erste Halteelement 29 auf. Dieses ist bei dem in Figur 2 dargestellten Ausführungsbeispiel als Lasche ausgebildet, welche in den Hohlraum 7 hineingebogen ist. Hier ist das erste Halteelement 29 einstückig mit dem Grundkörper 3 ausgebildet. Das erste Halteelement 29 kann beispielsweise mittels Einbringen eines U-förmigen Schnitts in den Grundkörpermantel 33, beispielsweise mittels Laserschneiden, realisiert, insbesondere aus dem Grundkörpermantel 33 herausgeschnitten werden. Das "U" des U-förmigen Schnitts in den Grundkörpermantel 33 öffnet sich hier in Richtung des proximalen Endes 15 des Grundkörpers 3.

Das erste Halteelement 29 bildet eine Lasche und ist derart in den Hohlraum 7 hineingebogen, dass sie im Wesentlichen orthogonal zu der Längsachse 5 angeordnet ist. Auf der dem distalen Ende 17 des Grundkörpers 3 zugewandten Oberfläche des ersten Halteelements 29 ist ein Anschlag 39 ausgebildet. Die Oberfläche selbst kann auch den Anschlag 39 bilden. Für eine sichere Funktion des Anschlags 39 reicht ein freies Ende 40 des ersten Halteelements 29 hier bis in einen Bereich der - aus Sicht des Betrachters - unteren Innenseite 9 des Grundkörpers 3. Ein größerer Abstand des freien Endes 40 des ersten Halteelements 29 kann jedoch ebenso ausreichend sein.

Der Anschlag 39 wirkt in der Betriebsposition 13 mit dem Drehkörper 11 derart zusammen, dass eine Verlagerung desselben in der Einführrichtung P_{E} über die Betriebsposition 13 hinaus verhindert wird. Das erste Halteelement 29 ist vorzugsweise starr, insbesondere nicht federnd ausgebildet. Dadurch kann eine vorbestimmte Begrenzung einer axialen Position des Drehkörpers 11 in der Einführrichtung P_{E} in der Betriebsposition 13 realisiert werden. Der Drehkörper 11 weist an seinem proximalen Ende 19 einen proximalen Abstützbereich 41 auf. Insbesondere kann der Drehkörper 11 so weit in den Hohlraum 7 eingeführt werden, bis der proximale Abstützbereich 41 an dem Anschlag 39 anliegt.

Die Halteeinrichtung 27 weist das wenigstens eine zweite Halteelement 31 auf. Bei dem in Figur 2 dargestellten Ausführungsbeispiel sind zwei zweite Halteelemente 31 vorgesehen, welche an zwei radial gegenüberliegenden Bereichen des Grundkörpermantels 33 angeordnet sind. Die zweiten Halteelemente 31 sind hier als rastnasenförmige Laschen ausgebildet, welche zumindest bereichsweise in den Hohlraum 7 hineinragen.

Die zweiten Halteelemente 31 können beispielsweise derart hergestellt werden, dass ein U-förmiger Schnitt in den Grundkörpermantel 33, beispielsweise mittels Laserschneiden, eingebracht wird. Das "U" des U-förmigen Schnitts öffnet sich hier in Richtung des distalen Endes 17 des Grundkörpers 3. Die dabei gebildeten Laschen werden dann vorzugsweise in den Hohlraum 7 hineingebogen. Die zweiten Halteelemente 31 sind hier einstückig mit dem Grundkörper 3 ausgebildet. Sie sind insbesondere an einem Anlenkbereich 43 mit dem Grundkörpermantel 33 verbunden. Insbesondere entspringen die zweiten Halteelemente 31 von dem Grundkörpermantel 33 an dem Anlenkbereich 43. Der Anlenkbereich 43 ist somit insbesondere ein Bereich des Grundkörpers 3, welcher biegeflexibel ausgebildet ist. Die die zweiten Halteelemente 31 bildenden rastnasenförmigen Laschen weisen mit einem freien Ende 44 im Wesentlichen in Richtung des proximalen Endes 15 des Grundkörpers 3. Die zweiten Halteelemente 31 sind vorzugsweise in einem flachen Winkel zu der Längsachse 5 angeordnet.

Die zweiten Halteelemente 31 weisen auf einer dem Hohlraum 7 zugewandten Seite einen Kontaktbereich 45 auf. Der Kontaktbereich 45 ist hier ein Teil der Innenseite 9 des Grundkörpers 3, welcher in den Hohlraum 7 hineinragt. Im Bereich des Kontaktbereichs 45 verjüngt sich also der Hohlraum 7 zumindest bereichsweise in Richtung der Einführrichtung P_{E}.

Die zweiten Halteelemente 31 weisen an ihrem freien Ende 44 einen Haltebereich 47 auf. Der Haltebereich 47 ist eingerichtet, den Drehkörper 11 in der Betriebsposition 13 an dem distalen Abstützbereich 25 zu hintergreifen. Auf diese Weise wird eine Rastverbindung zwischen Grundkörper 3 und Drehkörper 11 derart geschaffen, dass der Drehkörper 11 sicher in der Betriebsposition 13 gehalten wird. Ein Außendurchmesser des Schafts 23 ist insbesondere nicht zu groß ausgelegt, so dass ein derartiger Hintergriff möglich ist. Der Haltebereich 47 liegt insbesondere in einem Bereich, welcher einen radialen Abstand von der Längsachse 5 aufweist, welcher geringer ist, als ein radialer Abstand einer Mantelfläche 49 des Drehkörpers 11.

In der Betriebsposition 13 wirkt der Drehkörper 11 derart mit den zweiten Halteelementen 31 zusammen, dass der Drehkörper 11 an dem distalen Abstützbereich 25 durch den Haltebereich 47 der zweiten Halteelemente 31 in axialer Richtung gehalten wird, so dass eine Verlagerung des Drehkörpers 11 entgegen der Einführrichtung P_{E} verhindert ist.

Die zweiten Halteelemente 31 werden durch eine Rückstellkraft in einer Haltestellung gehalten, wobei sie, wie in Figur 2 dargestellt, in den Hohlraum 7 hineingebogen sind. Bei dem in Figur 2 dargestellten Ausführungsbeispiel ist die Rückstellkraft insbesondere eine Biegekraft, welche zum Biegen der zweiten Halteelemente 31 in radialer Richtung nach außen erforderlich ist. Es ist also insbesondere nicht möglich, den Drehkörper 11 aus der Betriebsposition 13 entgegen der Einführrichtung P_{E} aus dem Grundkörper 3 herauszuziehen.

Im Folgenden ist der Übergang von dem in den Figuren 1a), 1b) und 1c) dargestellten entkoppelten Zustand in den in Figur 2 dargestellten gekoppelten Zustand beschrieben: Ein Koppeln der Kupplung 1 wird durchgeführt, indem der Drehkörper 11 entlang der Einführrichtung P_{E} über das distale Ende 17 des Grundkörpers 3 in den Hohlraum 7 eingeführt wird. An dem proximalen Ende 19 des Drehkörpers 11 ist ein Angriffsbereich 51 vorgesehen. Der Angriffsbereich 51 tritt beim Einführen des Drehkörpers 11 in den Grundkörper 3 an einer bestimmten axialen Position in Kontakt mit dem Kontaktbereich 45 der zweiten Halteelemente 31. Der Drehkörper 11 wirkt mit dem Angriffsbereich 51 derart mit dem Kontaktbereich 45 zusammen, dass die zweiten Halteelemente 31 radial nach außen verlagert werden, mithin in eine Freigabestellung verlagert werden. Die Verlagerung der zweiten Halteelemente 31 in die Freigabestellung erfolgt gegen die Rückstellkraft. Die zweiten Halteelemente 31 werden insbesondere mittels des Angriffsbereichs 51 so weit radial nach außen verlagert, dass der lichte Querschnitt des Hohlraums 7 im Bereich der zweiten Halteelemente 31, insbesondere des Kontaktbereichs 45, ein Einführen des Drehkörpers 11 in die Betriebsposition 13 innerhalb des Grundkörpers 3 erlaubt. Der Drehkörper 11 ist so weit in den Hohlraum 7 einführbar, bis der proximale Abstützbereich 41 an dem Anschlag 39 anliegt. In dieser Position des Drehkörpers 11 bewegen sich die zweiten Halteelemente 31 aufgrund ihrer Rückstellkraft zur Realisierung der Verrastung nach innen in den Hohlraum 7 hinein.

In der Betriebsposition 13 ist der Drehkörper 11 vorzugsweise derart formschlüssig mit dem Grundkörper 3 gekoppelt, dass eine Drehung des Drehkörpers 11 relativ zu dem Grundkörper 3 um die Längsachse 5 bei möglichst geringer Reibung möglich ist. Besonders bevorzugt ist hierzu eine spielarme, vorzugsweise spielfreie Lagerung des Drehkörpers 11 in dem Grundkörper 3 in der Betriebsposition 13 vorgesehen. Besonders bevorzugt ist in der Betriebsposition 13 ein axialer Abstand zwischen dem Anschlag 39 und dem proximalen Abstützbereich 41 vorgesehen, welcher von wenigstens 0,1 mm bis höchstens 0,2 mm beträgt. Besonders bevorzugt ist in der Betriebsposition 13 zwischen dem distalen Abstützbereich 25 und dem Haltebereich 47 ein axialer Abstand vorgesehen, welcher von wenigstens 0,1 mm bis höchstens 0,2 mm beträgt. Besonders bevorzugt ist in der Betriebsposition 13 zwischen der Mantelfläche 49 des Drehkörpers 11 und der Innenseite 9 des Grundkörpers 3 ein radialer Abstand vorgesehen, welcher von wenigstens 0,03 mm bis höchstens 0,07 mm, vorzugsweise 0,05 mm beträgt. Vorzugsweise weist die Mantelfläche 49 des Drehkörpers 11 einen Außendurchmesser von 1,2 mm auf, wobei die Innenseite 9 des Grundkörpers 3 einen Innendurchmesser von 1,3 mm aufweist.

Die Halteeinrichtung 27 ist also eingerichtet, um vorzugsweise eine Verlagerung des Drehkörpers 11 relativ zu dem Grundkörper 3 entlang der Längsachse P_{E} sicher zu blockieren. Sie ist vorzugsweise auch eingerichtet, um eine Verlagerung des Drehkörpers 11 relativ zu dem Grundkörper 3 entlang der Längsachse 5 insbesondere entgegen der Einführrichtung P_{E} bei Bedarf freizugeben, worauf im Folgenden noch eingegangen wird.

Die Kupplung 1 zeichnet sich vorzugsweise dadurch aus, dass der Grundkörper 3 und der Drehkörper 11 elektrisch miteinander verbunden sind. Insbesondere durch die Lagerung des Drehkörpers 11 in dem Grundkörper 3 in der Betriebsposition 13 kann vorzugsweise sichergestellt werden, dass eine elektrische Verbindung zwischen dem Grundkörper 3 und dem Drehkörper 11 bei bestimmungsgemäßem Gebrauch des endoskopischen HF-chirurgischen Instruments sichergestellt ist.

Darüber hinaus sind vorzugsweise der Drehkörper 11 und der Schaft 23 elektrisch miteinander verbunden. Vorzugsweise ist der Schaft 23 mit dem Schneidinstrument, besonders bevorzugt einem HF-Schneidinstrument gekoppelt, wobei dann insbesondere der Drehkörper 11 und das Schneidinstrument elektrisch miteinander verbunden sind.

Der Grundkörper 3 kann vorzugsweise mit einer Stromquelle elektrisch verbunden werden. Vorzugsweise ist ein Stromleiter an einem Schlitz 53 des Grundkörpers 3 angeordnet und elektrisch mit dem Grundkörper 3 verbunden.

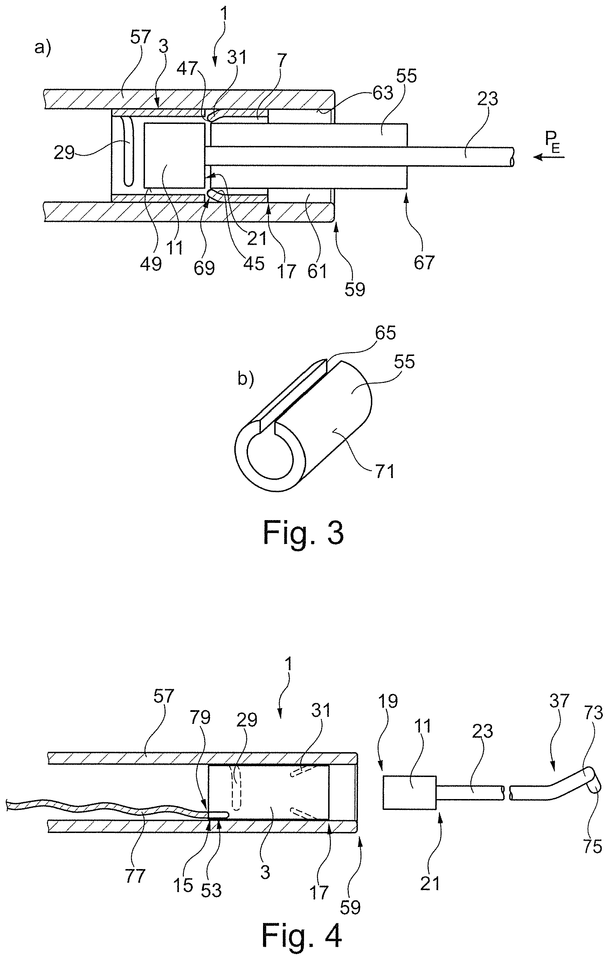

Figur 3a) zeigt eine schematische Darstellung eines Ausführungsbeispiels der Kupplung 1 mit einem Lösemittel 55 in einem Längsschnitt. Figur 3b) zeigt eine schematische Darstellung des Lösemittels 55 in einer perspektivischen Darstellung. Dabei ist in Figur 3a) das Lösemittel 55 in einer Draufsicht - aus Sicht des Betrachters - von oben auf das in Figur 3b) dargestellte Lösemittel 55 dargestellt. Gleiche und funktionsgleiche Elemente sind mit gleichen Bezugszeichen versehen, sodass insofern auf die vorangegangene Beschreibung verwiesen wird.

In Figur 3a) ist der Grundkörper 3 in einem Katheter 57 angeordnet. Der Katheter 57 weist ein distales Ende 59 auf. Der Grundkörper 3 ist in dem Katheter 57 verschiebesicher und vorzugsweise verdrehsicher angeordnet. Das distale Ende 17 des Grundkörpers 3 ist vorzugsweise in einem Abstand zu dem distalen Ende 59 des Katheters 57 angeordnet, wobei das distale Ende 59 über das distale Ende 17 hinauskragt. Es ergibt sich also ein Innenraum 61 zwischen dem distalen Ende 17 des Grundkörpers 3 und dem distalen Ende 59 des Katheters 57, welcher in radialer Richtung von einer Innenseite 63 des Katheters 57 begrenzt wird.

Die Kupplung 1 zeichnet sich vorzugsweise dadurch aus, dass eine Verlagerung der zweiten Halteelemente 31 in die Freigabestellung durch das Lösemittel 55 bewirkbar ist. Dann kann der Drehkörper 11 insbesondere aus der Betriebsposition 13 entgegen der Einführrichtung P_{E} verlagert werden.

Das Lösemittel 55 gemäß Figur 3 ist als zylindrische Hülse, insbesondere aus einem Rohrelement, ausgeführt, welche einen sich entlang einer Längsachse der Hülse erstreckenden Schlitz 65 aufweist. Der Schlitz 65 weist vorzugsweise eine Breite auf, welche wenigstens dem Durchmesser des Schafts 23 entspricht. Insbesondere ist der Schaft 23 derart auf das Lösemittel 55 abgestimmt, dass das Lösemittel 55 insbesondere außerhalb des Innenraums 61 auf den Schaft 23 aufgesetzt werden kann, so dass das Lösemittel 55 den Schaft 23 zumindest bereichsweise radial umgreift. Ein Außendurchmesser des Lösemittels 55 ist hier im Wesentlichen gleich groß wie der Außendurchmesser des Drehkörpers 11.

Eine Länge des Lösemittels 55 ist hier vorzugsweise so gewählt, dass das Lösemittel 55 auch bei maximal - aus Sicht des Betrachters - nach links verlagerter Position von einem Benutzer an einem distalen Bereich 67 desselben noch sicher gegriffen werden kann.

Die Verlagerung der zweiten Halteelemente 31 in die Freigabestellung ist durch Einführen des Lösemittels 55 in den Hohlraum 7 bewirkbar. Hierfür ist das Lösemittel 55 zunächst auf den Schaft 23 aufzusetzen und in Einführrichtung P_{E} zunächst in den Innenraum 61 und weiter in den Hohlraum 7 einzuführen. Ein proximaler Bereich 69 des Lösemittels 55 gerät bei einer bestimmten axialen Position des Lösemittels 55 in Kontakt mit den zweiten Halteelementen 31 insbesondere im Bereich des Kontaktbereichs 45. Wird das Lösemittel 55 weiter in Einführrichtung P_{E} auf den Drehkörper 11 zubewegt, werden die zweiten Halteelemente 31 von der Haltestellung radial nach außen in die Freigabestellung verlagert. In einer axialen Position des Lösemittels 55, in der der proximale Bereich 69 des Lösemittels 55 vorzugsweise das distale Ende 21 des Drehkörpers 11 berührt, sind die zweiten Halteelemente 31 in die Freigabestellung verlagert. Bei dem in Figur 3 dargestellten Ausführungsbeispiel bildet dann eine Mantelfläche 71 des Lösemittels 55 mit der Mantelfläche 49 des Drehkörpers 11 eine im Wesentlichen durchgehende zylindrische Mantelfläche. Es ist dann vorzugsweise möglich, den Drehkörper 11 zusammen mit dem Lösemittel 55 insbesondere durch Herausziehen des Schafts 23 entgegen der Einführrichtung P_{E} aus dem Grundkörper 3 zu verlagern. Es kann somit mittels des Lösemittels 55 insbesondere eine Verlagerung des Drehkörpers 11 relativ zu dem Grundkörper 3 entgegen der Einführrichtung P_{E} freigegeben werden.

Figur 3 zeigt somit auch einen Teil eines erfindungsgemäßen HF-chirurgischen Instruments, welches eine Kupplung 1 der beschriebenen Art aufweist. Das HF-chirurgische Instrument ist vorzugsweise flexibel ausgebildet. Es weist einen Katheter 57 auf, in welchem die Kupplung 1 verschiebesicher, gegebenenfalls auch verdrehsicher angeordnet ist. Der Katheter 57 weist ein distales Ende 59 auf, in dessen Bereich die Kupplung 1 angeordnet ist.

Figur 4 zeigt eine schematische Darstellung eines ersten Ausführungsbeispiels der Kupplung 1 mit einem Stromleiter in einem Längsschnitt. Gleiche und funktionsgleiche Elemente sind mit gleichen Bezugszeichen versehen, sodass insofern auf die vorangegangene Beschreibung verwiesen wird.

Bei diesem Ausführungsbeispiel ist schematisch das Schneidinstrument 73 dargestellt, welches vorzugsweise als Schneidnadel, besonders bevorzugt als HF-Schneidnadel ausgebildet ist. Das Schneidinstrument 73 ist an dem distalen Ende 37 des Schafts 23 angeordnet. Es ist möglich, dass der Schaft 23 und das Schneidinstrument 73 einstückig ausgebildet sind. Vorzugsweise weist das Schneidinstrument 73 eine Schneid-/Koagulationselektrode 75 auf.

Der vorzugsweise isolierend ausgebildete Katheter 57 weist einen Stromleiter 77 auf, welcher mit dem Grundkörper 3 elektrisch verbunden ist. Hier ist der Stromleiter 77 drahtförmig, vorzugsweise litzenförmig ausgebildet. Der drahtförmige Stromleiter 77 ist an einem Ende 79 desselben, vorzugsweise an dem Schlitz 53 des Grundkörpers 3 elektrisch mit dem Grundkörper 3 verbunden. Der drahtförmige Stromleiter 77 ist vorzugsweise an einem, dem Ende 79 entgegengesetzten Ende desselben mit einer Stromquelle verbunden. Es ist also möglich, über den drahtförmigen Stromleiter 77 den Grundkörper 3 mit einer Stromquelle elektrisch zu verbinden. Vorzugsweise ist der Grundkörper 3 mit dem Drehkörper 11 in der Betriebsposition 13 elektrisch verbunden, wobei bevorzugt der Drehkörper 11 und das Schneidinstrument 73 elektrisch miteinander verbunden sind.

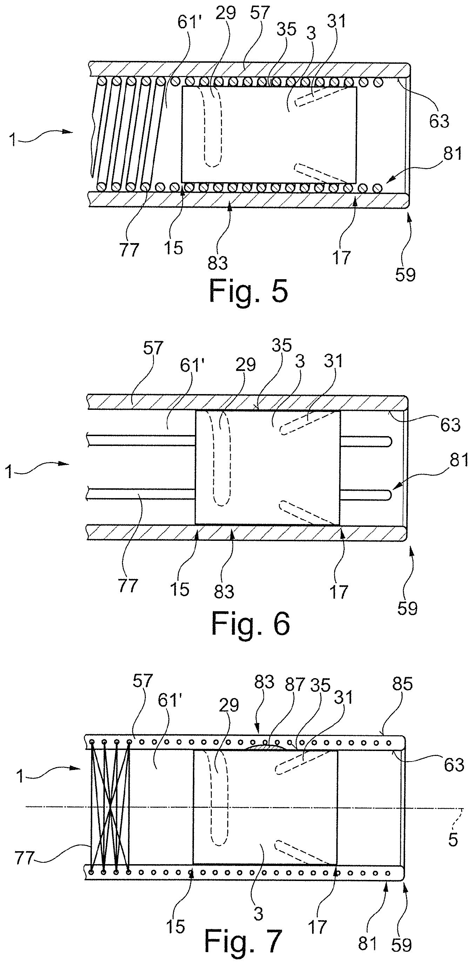

Figur 5 zeigt eine schematische Darstellung eines zweiten Ausführungsbeispiels der Kupplung 1 mit einem Stromleiter 77 in einem Längsschnitt. Gleiche und funktionsgleiche Elemente sind mit gleichen Bezugszeichen versehen, sodass insofern auf die vorangegangene Beschreibung verwiesen wird.

Der vorzugsweise isolierend ausgebildete Katheter 57 weist hier einen Stromleiter 77 auf, mit welchem der Grundkörper 3 elektrisch verbunden ist. Der Stromleiter 77 ist hier schraubenfederförmig ausgebildet. Der schraubenfederförmige Stromleiter 77 ist an der Innenseite 63 des Katheters 57 angeordnet. Das distale Ende 81 des schraubenfederförmigen Stromleiters 77 liegt hier im Bereich des distalen Endes 59 des Katheters 57.

Ein durch den schraubenfederförmigen Stromleiter 77 radial begrenzter Innenraum 61' des Katheters 57 ist insbesondere radial auf den Außendurchmesser des Grundkörpers 3 derart abgestimmt, dass der Grundkörper 3 verschiebesicher, gegebenenfalls auch verdrehsicher in dem Katheter 57 anordenbar ist. Ebenso ist vorzugsweise der schraubenfederförmige Stromleiter 77 verdreh- und verschiebesicher an der Innenseite 63 des Katheters 57 angeordnet. Entscheidend ist dabei, dass der Grundkörper 3 in dem Katheter 57 insbesondere verschiebesicher und gegebenenfalls auch verdrehsicher relativ zu dem Katheter 57 angeordnet ist.

Der schraubenfederförmige Stromleiter 77 ist in einem Kontaktbereich 83, welcher hier einen Abstand von dem distalen Ende 81 des schraubenfederförmigen Stromleiters 77 aufweist, mit dem Grundkörper 3 elektrisch verbunden. Es wird bevorzugt, dass der Grundkörper 3 auf der Außenseite 35 zumindest bereichsweise elektrisch leitendes Material aufweist oder aus elektrisch leitendem Material besteht, sodass durch Kontakt desselben mit dem schraubenfederförmigen Stromleiter 77 eine elektrische Verbindung zwischen dem schraubenfederförmigen Stromleiter 77 und dem Grundkörper 3 hergestellt wird. Der schraubenfederförmige Stromleiter 77 ist vorzugsweise an einem, dem distalen Ende 81 entgegengesetzten Ende desselben mit einer Stromquelle elektrisch verbunden.

Insbesondere bei der Montage des Grundkörpers 3 in dem Katheter 57 ergeben sich Vorteile aus dieser Anordnung, da eine elektrische Verbindung des Grundkörpers 3 mit dem schraubenfederförmigen Stromleiter 77 vorzugsweise in einem bestimmten Bereich unabhängig von einer axialen Position des Grundkörpers 3 relativ zu dem Katheter 57 ist. Hierdurch wird eine Montage des Grundkörpers 3 in dem Katheter 57 vereinfacht, wodurch Kosten gesenkt werden können.

Figur 6 zeigt eine schematische Darstellung eines dritten Ausführungsbeispiels der Kupplung 1 mit einem Stromleiter 77 in einem Längsschnitt. Gleiche und funktionsgleiche Elemente sind mit gleichen Bezugszeichen versehen, sodass insofern auf die vorangegangene Beschreibung verwiesen wird.

Der Stromleiter 77 ist hier bandförmig ausgebildet. Hier sind zwei bandförmige Stromleiter 77 dargestellt. Es ist insbesondere möglich, dass eine beliebige Anzahl von bandförmigen Stromleitern 77 vorgesehen ist. Die bandförmigen Stromleiter 77 sind vorzugsweise an der Innenseite 63 des vorzugsweise isolierend ausgebildeten Katheters 57 angeordnet. Vorzugsweise sind die bandförmigen Stromleiter 77 in die Innenseite 63 des Katheters 57 eingelassen. Besonders bevorzugt weisen die bandförmigen Stromleiter 77 eine Metallfolie auf oder bestehen aus einer Metallfolie. Es ist aber auch möglich, dass die bandförmigen Stromleiter 77 elektrisch leitenden Kunststoff aufweisen oder aus elektrisch leitendem Kunststoff bestehen.

Die bandförmigen Stromleiter 77 erstrecken sich hier in Längsrichtung entlang des Katheters 57. Das distale Ende 81 der bandförmigen Stromleiter 77 liegt hier im Bereich des distalen Endes 59 des Katheters 57. Der Grundkörper 3 ist in dem Kontaktbereich 83, welcher hier einen Abstand von dem distalen Ende 81 des bandförmigen Stromleiters 77 aufweist, elektrisch mit dem bandförmigen Stromleiter 77 verbunden. Eine elektrische Verbindung zwischen dem Grundkörper 3 und dem bandförmigen Stromleiter 77 ist vorzugsweise durch einen zumindest bereichsweise elektrisch leitenden Bereich auf der Außenseite 35 des Grundkörpers 3 realisiert, welcher den bandförmigen Stromleiter 77 berührt.

Insbesondere bei der Montage des Grundkörpers 3 in dem Katheter 57 ergeben sich Vorteile aus dieser Anordnung, da eine elektrische Verbindung des Grundkörpers 3 mit dem bandförmigen Stromleiter 77 vorzugsweise in einem bestimmten Bereich unabhängig von einer axialen Position des Grundkörpers 3 relativ zu dem Katheter 57 ist. Hierdurch wird eine Montage des Grundkörpers 3 in dem Katheter 57 vereinfacht, wodurch Kosten gesenkt werden können.

Figur 7 zeigt eine schematische Darstellung eines vierten Ausführungsbeispiels der Kupplung 1 mit einem Stromleiter 77 in einem Längsschnitt. Gleiche und funktionsgleiche Elemente sind mit gleichen Bezugszeichen versehen, sodass insofern auf die vorangegangene Beschreibung verwiesen wird.

Der Stromleiter 77 ist hier geflechtartig ausgebildet. Der Übersichtlichkeit wegen ist in Figur 7 nur ein Teil des Geflechts dargestellt. Der geflechtartige Stromleiter 77 ist vorzugsweise innerhalb des Katheters 57, insbesondere zwischen der Innenseite 63 des Katheters 57 und einer Außenseite 85 des Katheters 57 angeordnet. Durch die isolierende Ausgestaltung des Katheters 57 ist eine elektrische Isolation insbesondere in Richtung des Innenraums 61' und der Außenseite 85 realisiert. Der geflechtartige Stromleiter 77 ist hier quasi rohrförmig in dem Katheter 57 ausgebildet. Er erstreckt sich bis zu dem distalen Ende 81, welches im Bereich des distalen Endes 59 des Katheters 57 liegt.

Der Grundkörper 3 weist an der Außenseite 35 eine Kante 87 auf. Diese erstreckt sich vorzugsweise in einem axialen Abschnitt der Außenseite 35 des Grundkörpers 3 entlang der Längsachse 5. Die Kante 87 ist vorzugsweise scharf ausgebildet. Hierdurch ist es möglich, insbesondere bei der Montage des Grundkörpers 3 in dem Katheter 57 durch radiales Hineindrücken der Kante 57 in die Innenseite 63 des Katheters 57 einen Schnitt in die Innenseite 63 einzubringen, sodass die Kante 57 den geflechtartigen Stromleiter 77 berühren kann. Die Kante 87 ist zumindest bereichsweise elektrisch leitend ausgebildet, sodass eine elektrische Verbindung der Kante 57 mit dem geflechtartigen Stromleiter 77 herstellbar ist. Es ist also möglich, eine elektrische Verbindung des geflechtartigen Stromleiters 77 mit dem Grundkörper 3 über die Kante 87 zu realisieren.

Es erweist sich als vorteilhaft, dass die elektrische Verbindbarkeit des Grundkörpers 3 mit dem geflechtartigen Stromleiter 77 insbesondere in einem bestimmten Bereich unabhängig von einer axialen Anordnung des Grundkörpers 3 relativ zu dem Katheter 57 realisierbar ist. Hierdurch wird eine Montage des Grundkörpers 3 in dem Katheter 57 vereinfacht, wodurch Kosten gesenkt werden können.

Aus den Figuren 3 bis 7 ist ersichtlich, dass der Grundkörper 3 in einem Abstand zum distalen Ende des Katheters 57 in dessen Innenraum angeordnet ist. Auf diese Weise wird sichergestellt, dass Gewebe eines Patienten oder des Benutzers des Katheters 57 nicht mit stromführenden Teilen in Berührung kommt. Weiterhin ist aus den Figuren 3 bis 7 ersichtlich, dass der Stromleiter 77 nicht über einen, in proximaler Richtung von dem distalen Ende 59 des Katheters 57 beabstandeten Bereich in distaler Richtung hinausgeführt wird. Insbesondere endet der Stromleiter 77 an diesem - in den Figuren 5 bis 7 mit dem Bezugszeichen 81 gekennzeichneten - Bereich, also in einem Abstand zum distalen Ende 59. Auch damit wird sichergestellt, dass Gewebe des Patienten oder des Benutzers des Katheters 57 nicht mit dem Stromleiter 77 in Berührung kommt.

Insgesamt zeigt sich, dass durch die Kupplung 1 auf einfache Weise eine Verbindung zwischen einem Grundkörper 3 und einem Drehkörper 11 hergestellt werden kann, insbesondere weil der Grundkörper 3 eine einfach zu realisierende Halteeinrichtung 27 mit mindestens einem zweiten Halteelement 31 umfasst, welches nach dem Einführen des Drehkörpers 11 in den Grundkörper 3 hinter diesem verrastend einschnappt. Dadurch wird mit höchster Sicherheit verhindert, dass der Drehkörper 11 und damit ein chirurgisches Schneidinstrument sich bei einer Operation von dem Grundkörper 3 und einem damit verbundenen Katheter 57 löst. Dies führt zu einer hohen gesundheitlichen Sicherheit für Patienten, bei denen das chirurgische Schneidinstrument und insbesondere die hier beschriebene Kupplung 1 eingesetzt werden.

Bei dieser Ausgestaltung ist auch ein Wechsel eines drehbeweglich gelagerten Schneidinstruments 73 durch Kopplung und Entkopplung des Grundkörpers 3 und des Drehkörpers 11 mit Hilfe eines Lösemittels 55 auf sichere und einfache Weise möglich. Es ist damit möglich, vor einer Operation ein beispielsweise längenmäßig geeignetes Schneidinstrument 73 mit dem Katheter 57 zu koppeln. Ebenso ist es möglich, während einer Operation einen einfachen und schnellen Austausch des Schneidinstruments 73 vorzunehmen. Neben einer erhöhten Anwendungsflexibilität beim Betrieb des Instruments ergeben sich zudem Kosteneinsparungen beispielsweise durch eine mehrfache Verwendung des Katheters.

## Patentansprüche

1. Kupplung (1) eines endoskopischen HF-chirurgischen Instruments zum Koppeln eines Schneidinstruments (73) mit einem Katheter (57), mit
- einem Grundkörper (3), welcher
- einen sich entlang einer Längsachse (5) des Grundkörpers (3) erstreckenden Hohlraum (7) aufweist, in welchen
- ein Drehkörper (11) bis zu einer Betriebsposition (13) in einer Einführrichtung (P_{E}) einführbar ist, wobei
- der Drehkörper (11) in der Betriebsposition (13) relativ zu dem Grundkörper (3) um die Längsachse (5) drehbar ist, wobei
- der Grundkörper (3) wenigstens eine Halteeinrichtung (27) aufweist, welche
- eingerichtet ist, um den Drehkörper (11) in der Betriebsposition (13) in dem Grundkörper (3) formschlüssig derart zu verrasten, dass
- eine Verlagerung des Drehkörpers (11) relativ zu dem Grundkörper (3) entlang der Längsachse (5) blockierbar ist
**dadurch gekennzeichnet, dass**
a) die Halteeinrichtung (27) ein erstes Halteelement (29) aufweist, welches in dem Hohlraum (7) einen Anschlag (39) ausbildet, wobei der Anschlag (39) in der Betriebsposition (13) mit dem Drehkörper (11) derart zusammenwirkt, dass eine Verlagerung desselben in der Einführrichtung (P_{E}) über die Betriebsposition (13) hinaus verhindert wird, wobei das erste Halteelement (29) einstückig mit dem Grundkörper (3) ausgebildet ist, und/oder dass
b) die Halteeinrichtung (27) wenigstens ein flexibles zweites Halteelement (31) aufweist, welches zum Einführen des Drehkörpers (11) in den Hohlraum (7) gegen eine Rückstellkraft in eine Freigabestellung verlagerbar ist, wobei das wenigstens eine flexible zweite Halteelement (31) einstückig mit dem Grundkörper (3) ausgebildet ist.

2. Kupplung (1) nach Anspruch 1, mit dem Drehkörper (11), **dadurch gekennzeichnet, dass** der Drehkörper (11) in der Betriebsposition (13) gänzlich in dem Hohlraum (7) angeordnet ist.

3. Kupplung (1) nach einem der vorhergehenden Ansprüche, **dadurch gekennzeichnet, dass** die Rückstellkraft eine Verlagerung des wenigstens einen zweiten Halteelements (31) in eine Haltestellung bewirkt, wenn sich der Drehkörper (11) in der Betriebsposition (13) befindet, wodurch eine Verlagerung des Drehkörpers (11) entgegen der Einführrichtung (P_{E}) verhindert ist.

4. Kupplung (1) nach einem der vorhergehenden Ansprüche, **dadurch gekennzeichnet, dass** der Grundkörper (3) einstückig, vorzugsweise als hülsenförmiger Hohlzylinder, vorzugsweise aus einem nahtlosen Rohrelement, ausgebildet ist.

5. Kupplung (1) nach einem der vorhergehenden Ansprüche, **dadurch gekennzeichnet, dass**
a) das erste Halteelement (29) als Lasche ausgebildet ist, und/oder dass
b) das wenigstens eine flexible zweite Halteelement (31) als bevorzugt rastnasenförmige Lasche ausgebildet ist.

6. Kupplung (1) nach einem der vorhergehenden Ansprüche, **dadurch gekennzeichnet, dass** eine Verlagerung des wenigstens einen zweiten Halteelements (31) in die Freigabestellung durch ein Lösemittel (55) bewirkbar ist, wodurch der Drehkörper (11) aus der Betriebsposition (13) entgegen der Einführrichtung (P_{E}) verlagert werden kann, wobei vorzugsweise die Verlagerung des wenigstens einen zweiten Halteelements (31) in die Freigabestellung durch Einführen des Lösemittels (55) in den Hohlraum (7) bewirkbar ist.

7. Kupplung (1) nach einem der vorhergehenden Ansprüche, **dadurch gekennzeichnet, dass** der Grundkörper (3) und der Drehkörper (11), und/oder der Drehkörper (11) und das Schneidinstrument (73) elektrisch miteinander verbunden sind.

8. Kupplung (1) nach einem der vorhergehenden Ansprüche, **dadurch gekennzeichnet, dass** die Halteeinrichtung (27) des Grundkörpers (3) so ausgebildet ist, dass eine Verlagerung des Drehkörpers (11) relativ zu dem Grundkörper (3) entlang der Längsachse (5) freigebbar ist.

9. HF-chirurgisches Instrument, **gekennzeichnet durch** eine Kupplung (1) nach einem der Ansprüche 1 bis 8.

10. HF-chirurgisches Instrument nach Anspruch 9, **dadurch gekennzeichnet, dass** das HF-chirurgische Instrument flexibel ausgebildet ist.

11. HF-chirurgisches Instrument nach einem der Ansprüche 9 oder 10 **gekennzeichnet durch** einen Katheter (57), in welchem die Kupplung (1) verschiebesicher anordenbar ist.

12. HF-chirurgisches Instrument nach Anspruch 11, **dadurch gekennzeichnet, dass** der Katheter (57) ein distales Ende (59) aufweist, in dessen Bereich der Grundkörper (3) der Kupplung (1) insbesondere verschiebesicher und/oder verdrehsicher angeordnet ist.

13. HF-chirurgisches Instrument nach Anspruch 12, **dadurch gekennzeichnet, dass** der Grundkörper (3) in den Katheter (57) eingepresst ist.

## Claims

1. A coupling (1) of an endoscopic HF surgical instrument for coupling a cutting instrument (73) with a catheter (57), with
- a base body (3) that
- presents a cavity (7) extending along a longitudinal axis (5) of the base body (3) into which
- a rotating body (11) can be inserted in a direction of insertion (P_{E}) up to an operational position (13), wherein
- in the operational position (13), the rotating body (11) is rotatable relative to the base body (3) about the longitudinal axis (5), wherein
- the base body (3) has at least one retaining device (27) which
- is configured to lock the rotating body (11) in a form-fitting manner in the operational position (13) in the base body (3) such that
- a displacement of the rotating body (11) relative to the base body (3) along the longitudinal axis (5) can be blocked,
**characterized in that**
a) the retaining device (27) has a first retaining element (29) forming a stop (39) in the cavity (7), wherein in the operational position (13) the stop (39) cooperates with the rotating body (11) in such a manner that a displacement thereof in the direction of insertion (P_{E}) beyond the operational position (13) is prevented, wherein the first retaining element (29) is formed in one piece with the base body (3), and/or that
b) the retaining device (27) has at least one flexible second retaining element (31) which is displaceable into a release position against a resetting force for insertion of the rotating body (11) into the cavity (7), wherein the at least one flexible second retaining element (31) is formed in one piece with the base body (3).

2. The coupling (1) according to claim 1 with the rotating body (11), **characterised in that** in the operational position (13) the rotating body (11) as a whole is arranged in the cavity (7).

3. The coupling (1) according to any one of the preceding claims, **characterised in that** the resetting force effects a displacement of the at least one second retaining element (31) into a detent position when the rotating body (11) is in the operational position (13), thereby preventing a displacement of the rotating body (11) against the direction of insertion (P_{E}).

4. The coupling (1) according to any one of the preceding claims, **characterised in that** the base body (3) is configured in one piece, preferably as a hollow cylinder in the form of a sleeve, preferably made of a seamless tube element.

5. The coupling (1) according to any one of the preceding claims, **characterised in that**
a) the first retaining element (29) is configured as a lug, and/or that
b) the at least one flexible second retaining element (31) is configured as a lug, preferably in the form of a snap-in detent.

6. The coupling (1) according to any one of the preceding claims, **characterised in that** a displacement of the at least one second retaining element (31) into the release position can be effected by means of a releasing means (55), whereby the rotating body (11) can be displaced from the operational position (13) against the direction of insertion (P_{E}), wherein the displacement of the at least one second retaining element (31) into the release position preferably can be effected by inserting the releasing means (55) into the cavity (7).

7. The coupling (1) according to any one of the preceding claims, **characterised in that** the base body (3) and the rotating body (11), and/or the rotating body (11) and the cutting instrument (73) are electrically connected to each other.

8. The coupling (1) according to any one of the preceding claims, **characterised in that** the retaining device (27) of the base body (3) is configured in such a way that a displacement of the rotating body (11) relative to the base body (3) along the longitudinal axis (5) can be released.

9. An HF surgical instrument, **characterized by** a coupling (1) according to any one of claims 1 to 8.

10. The HF surgical instrument according to claim 9, **characterised in that** the RF surgical instrument has a flexible configuration.

11. The HF surgical instrument according to any one of claims 9 or 10 **characterized by** a catheter (57) in which the coupling (1) can be arranged secured against displacement.

12. The HF surgical instrument according to claim 11, **characterised in that** the catheter (57) has a distal end (59) in the region of which the base body (3) of the coupling (1) can be arranged, in particular secured against displacement and/or against rotation.

13. The HF surgical instrument according to claim 12, **characterised in that** the base body (3) is press-fitted into the catheter (57).

## Revendications

1. Accouplement (1) d'un instrument chirurgical à haute fréquence endoscopique pour accoupler un instrument de coupe (73) avec un cathéter (57), avec
- un corps de base (3) qui
- a une cavité (7) s'étendant le long d'un axe longitudinal (5) du corps de base (3) et dans laquelle
- un corps rotatif (11) peut être inséré dans une direction d'insertion (P_{E}) jusqu'à une position de fonctionnement (13),
- le corps rotatif (11) pouvant être tourner, dans la position de fonctionnement (13), par rapport au corps de base (3) autour de l'axe longitudinal (5),
- le corps de base (3) présentant au moins un dispositif de rétention (27) qui
- est configuré pour verrouiller le corps rotatif (11) dans la position de fonctionnement (13) dans le corps de base (3) par engagement positif de telle manière que
- un déplacement du corps rotatif (11) par rapport au corps de base (3) le long de l'axe longitudinal (5) peut être bloqué,
**caractérisé en ce que**
a) le dispositif de rétention (27) a un premier élément de rétention (29) formant une butée (39) dans la cavité (7), la butée (39) coopérant avec le corps rotatif (11) dans la position de fonctionnement (13) de telle manière à empêcher son déplacement dans la direction d'insertion (P_{E}) au-delà de la position de fonctionnement (13), le premier élément de rétention (29) étant formé d'un seul tenant avec le corps de base (3), et/ou que
b) le dispositif de rétention (27) a au moins un second élément de rétention (31) flexible qui peut être déplacé dans une position de dégagement contre une force de rappel, pour l'insertion du corps rotatif (11) dans la cavité (7), l'au moins un second élément de rétention (31) flexible étant formé d'un seul tenant avec le corps de base (3).

2. Accouplement (1) selon la revendication 1 avec le corps rotatif (11), **caractérisé en ce que**, dans la position de fonctionnement (13), le corps rotatif (11) est disposé entièrement dans la cavité (7).

3. Accouplement (1) selon l'une quelconque des revendications précédentes, **caractérisé en ce que** la force de rappel effectue un déplacement de l'au moins un second élément de rétention (31) dans une position de rétention lorsque le corps rotatif (11) se trouve dans la position de fonctionnement (13), ainsi empêchant un déplacement du corps rotatif (11) contre la direction d'insertion (P_{E}).

4. Accouplement (1) selon l'une quelconque des revendications précédentes, **caractérisé en ce que** le corps de base (3) est formé d'un seul tenant, de préférence sous forme d'un cylindre creux en forme de manchon, de préférence d'un élément de tube sans soudure.

5. Accouplement (1) selon l'une quelconque des revendications précédentes, **caractérisé en ce que**
a) le premier élément de rétention (29) est configuré sous forme de languette, et/ou que
b) l'au moins un second élément de rétention (31) flexible est configuré sous forme de languette, de préférence en forme de nez de verrouillage.

6. Accouplement (1) selon l'une quelconque des revendications précédentes, **caractérisé en ce qu'**un déplacement de l'au moins un second élément de rétention (31) dans la position de dégagement peut être effectué par un moyen de libération (55), par lequel le corps rotatif (11) peut être déplacé de la position de fonctionnement (13) contre la direction d'insertion (P_{E}), le déplacement de l'au moins un second élément de rétention (31) dans la position de dégagement de préférence pouvant être effectué par insertion du moyen de libération (55) dans la cavité (7).

7. Accouplement (1) selon l'une quelconque des revendications précédentes, **caractérisé en ce que** le corps de base (3) et le corps rotatif (11), et/ou le corps rotatif (11) et l'instrument de coupe (73), sont électriquement reliés l'un à l'autre.

8. Accouplement (1) selon l'une quelconque des revendications précédentes, **caractérisé en ce que** le dispositif de rétention (27) du corps de base (3) est configuré de telle manière qu'un déplacement du corps rotatif (11) par rapport au corps de base (3) le long de l'axe longitudinal (5) peut être libéré.

9. Instrument chirurgical à haute fréquence, **caractérisé par** un accouplement (1) selon l'une quelconque des revendications 1 à 8.

10. Instrument chirurgical à haute fréquence selon la revendication 9, **caractérisé en ce que** l'instrument chirurgical à haute fréquence à une configuration flexible.

11. Instrument chirurgical à haute fréquence selon l'une quelconque des revendications 9 ou 10, **caractérisé par** un cathéter (57) dans lequel l'accouplement (1) peut être disposé de manière sécurisée contre les déplacements.

12. Instrument chirurgical à haute fréquence selon la revendication 11, **caractérisé en ce que** le cathéter (57) a une extrémité distale (59) au niveau de laquelle le corps de base (3) de l'accouplement (1) est disposé, de préférence de manière sécurisée contre les déplacements et/ou sécurisée contre rotation.

13. Instrument chirurgical à haute fréquence selon la revendication 12, **caractérisé en ce que** le corps de base (3) est pressé dans le cathéter (57).
